# EUROPEAN PATENT APPLICATION

(11) **EP 1 972 342 A1**
(43) Date of publication of application: **24.09.2008**
(21) Application number: 06807890.6
(22) Date of filing: 28.07.2006
(51) Int. Cl.: A61K 33/24, A61P 25/18, A61P 25/28

(54) **PHARMACEUTICAL COMPOSITIONS COMPRISING A TUNGSTEN SALT (VI) FOR THE TREATMENT OF NEURODEGENERATIVE DISORDERS, PARTICULARLY ALZHEIMER'S DISEASE AND SCHIZOPHRENIA**

(30) Priority: 29.07.2005 ES 200501941
(71) Applicant: Universidad de Barcelona, 08028 Barcelona (ES); CONSEJO SUPERIOR DE INVESTIGACIONES CIENTIFICAS, 28006 Madrid (ES)
(72) Inventor: ZAFRA LÓPEZ, Delia, E-430003 Tarragona (ES); COROMINOLA OCAÑA, Helena CBM-SO, Fac. de Ciencias, E-28049 Madrid (ES); GUINOVART CIRERA, Joan Josep, E-08019 Barcelona (ES); ÁVILA DE GRADO, Jesús, E-08021 Barcelona (ES); DOMÍNGUEZ REYES, Jorge CBM-SO, Fac. de Ciencias, E-28049 Barcelona (ES); GOMIS DE BARBARÁ, Ram n, E-08042 Barcelona (ES); GÓMEZ RAMOS, Alberto, E-28049 Madrid (ES)
(74) Representative: Barlocci, Anna
(86) International application number: PCT/ES2006/000442
(87) International publication number: WO 2007/014970

(57) **Abstract**

Pharmaceutical compositions comprising an effective amount of a tungsten (VI) compound, preferably of a tungstate salt, and more preferably of sodium tungstate (Na₂WO₄), are useful for the prophylactic and/or therapeutical treatment of neurodegenerative disorders in a mammal, including a human, in particular, for the prophylactic and/or therapeutical treatment of Alzheimer's disease or schizophrenia. The effect of sodium tungstate dihydrate on the phosphorylation of tau in a model of rat insulin resistance and in a model of type-1 diabetes has been assessed. The therapeutic treatment of tauopathies that derives from this invention involves several advantages: it targets a GSK3; specificity since it reduces the abnormal hyperphosphorylation of a neural specific protein, tau, efficacy, lack of toxicity, and low price.

## Description

The invention refers to the use of pharmaceutical compositions comprising tungsten (VI) compounds for the treatment of neurodegenerative disorders, in particular, Alzheimer's disease and other tauopathies, i.e. neurodegenerative disorders involving deposition of abnormal tau protein isoforms in brain, and also schizophrenia.

### BACKGROUND ART

Neurodegenerative disorders can be defined as chronic and progressive disorders of the nervous system affecting neurologic and behavioral functions, which start with specific biochemical changes that ultimately lead to distinct histopathologic and clinical syndromes. Among such disorders are Alzheimer's disease, Huntington's disease and Parkinson's disease.

Alzheimer's disease is the most common form of dementia among older people, which involves the parts of the brain that control thought, memory, and language. It is characterized by two main pathological hallmarks: amyloid plaques and neurofibrillary tangles (NFT), in addition to neuron cell loss in specific brain regions. β-amyloid is a protein fragment that is snipped off from a protein called amyloid precursor protein (APP). In a healthy brain, these protein fragments are broken down and eliminated. In Alzheimer's disease, β-amyloid accumulate to form hard insoluble plaques named amyloid plaques. NFTs consist of insoluble twisted fibers that are found inside of the brain's cells. They primarily consist of the neuronal specific protein called tau, which forms part of subcellular structures called microtubules. The microtubules are one of the components of the cellular cytoskeleton and are involved in several functions such as intracellular transport and the generation of asymmetry. The main function of tau protein in the healthy state is to stabilize the microtubules. In Alzheimer's disease and also in other tauopathies, the tau protein displays some biochemical aberrations, of which hyperphosphorylation is the most striking. As a consequence tau protein aggregates, the microtubules are destabilized and, consequently, the neuronal function is compromised.

Protein phosphorylation is a post-translational regulatory mechanism used by cells to modulate enzymes and structural proteins. The phosphorylation and de-phosphorylation of tau protein at threonine and serine residues is controlled by several protein kinases and protein phosphatases. One of the tau-phosphorylating protein kinases is glycogen synthase kinase-3 (GSK3). GSK3 is the major enzyme involved in the process of abnormal hyperphosphorylation of tau protein, and is over-expressed in the brains of patients with Alzheimer's disease as well as in patients with other tauopathies. It has been proposed that pharmacological inhibitors of the phosphorylation of tau protein, specifically selective GSK3 inhibitors, could be used to treat Alzheimer's disease and other neurogenerative diseases.

Several inhibitors of GSK3 are known, but the toxicity associated side-effects and concerns regarding the absorption, distribution, metabolism and excretion of the known inhibitors affect their clinical potential (cf. "Pharmacological inhibitors of glycogen synthase kinase 3", Laurent Meijer et al., Trends in Pharmacological Sciences 2004, vol. 25, No. 9, pp. 471-480).

Some of the most important keys to the development of inhibitors of GSK3 for the effective treatment of Alzheimer's disease and other tauopathies are the availability of selective inhibitors of this kinase with absence of interference with other cell signaling processes, meaning that they do not have adverse effects, ability to pass the blood-brain barrier, and potency in vivo.

Changes in regulation of GSK3 activity have also been associated with schizophrenia (cf. E.S: Emamian et al., Nat. Genet., 2004, vol. 36, pp.131-7). According to J.A. Lieberman, Schizophrenia clearlly fulfills many of the criteria that define neurodegenerative disorders (cf.J.A. Lieberman, Biological Psychiatry, 1999, vol. 46, pp. 729-739). Direct and indirect evidences that the progressive course of schizophrenia is associated with ongoing neurodegenerative processes have also been reported (cf. P.C. Ashe et al., Prog. Neuro-Biol. Psychiat. & Biol. Psychiat 2001, vol.25, pp.691-707).

Despite all the research efforts invested in the past, the treatment and/or prevention of neurodegenerative dissorders such as Alzheimer's disease or schizophrenia is far from being satisfactory. Therefore, the provision of compounds for the treatment of pathological alterations in neural systems related to phosphorylation of tau, such as Alzheimer's disease and other tauopathies, as well as for the treatment of schizophrenia, in humans, is of major importance.

### SUMMARY OF THE INVENTION

Inventors have found that tungsten (VI) compounds inhibit GSK3 in neural cells both in cell culture systems and in vivo. The main consequence of this inhibition is a significant reduction in the GSK3-dependent phosphorylation of the microtubule-associated protein tau. Thus, given that tungsten (VI) compounds contribute to the inactivation of GSK3, the use of these compounds represent a new therapeutical approach to treat Alzheimer's disease and other tauopathies, an also to treat schizophrenia.

Sodium tungstate is an anti-diabetic and anti-obesity agent in several animal models. This compound shows a low toxicity profile and Phase I of clinical trials has already been finished. EP 1.400.246-A describes a pharmaceutical composition of a tungsten (VI) compound for lowering glycemia in humans suffering from Type 1 (IDDM) or Type 2 (NIDDM) diabetes mellitus. EP 755.681-A teaches that tungsten (VI) compounds are also efficient compounds for the treatment of obesity/overweight in non-diabetic humans. Nevetheless, tungsten (VI) compounds have never been proposed for the treatment of Alzheimer's disease or any tauopathy, or schizophrenia.

According to an aspect of the present invention, it is provided a pharmaceutical composition for the prophylactic and/or therapeutic treatment of neurodegenerative disorders in a mammal, including a human, such as schizophrenia or tauopathies such as Alzheimer's disease, frontotemporal dementia with parkinsonism linked to chromosome 17, progressive supranuclear palsy, amyotrophic lateral sclerosis/parkinsonism-dementia complex of Guam, Pick's disease, corticobasal degeneration, and Argyrophilic grain disease, said composition comprising an effective amount of a compound formed by tungsten (VI) and a pharmaceutically acceptable chemical moiety, or of a solvate of said compound, in combination with pharmaceutically acceptable excipients or carriers.

In the context of this invention, the expression "a compound formed by tungsten (VI) and a pharmaceutically acceptable chemical moiety" is intended to include any chemical entity formed by one or several tungsten atoms in its 6+ oxidation state attached to a chemical structure that is pharmaceutically acceptable by itself. The cation W⁶⁺ has neither been observed nor isolated, and it comes always accompanied with a chemical moiety partially formed by a coordination sphere around the atom of W(VI). The coordination sphere can be formed by inorganic ligands (oxide, hydroxide, peroxide, phosphate etc.) as, for example, in the case of the tungstate anion (coordination sphere formed by four oxide ions), or in the case of peroxytungstates (coordination sphere formed by mixtures of oxide and peroxide ions). The coordination sphere can also be formed by organic ligands which are molecules or ions attached to W(VI) atom through O, S or N atoms belonging to different pharmaceutically acceptable organic compounds (e.g. pharmaceutically acceptable alcohols, thiols, carboxylic acids, amines, aminoacids, N-containing heterocycles, etc.). Mixed inorganic/organic coordination spheres are also possible. When the structure formed by the W(VI) atom and its coordination sphere is not neutral, the term "chemical moiety" also includes any pharmaceutically acceptable ionic species which makes neutral the whole tungsten (VI) compound. For example, the tungstate anion is always accompanied by a cation (e.g. sodium, potassium, magnesium, calcium) to form a neutral tungstate salt. Tungstate ion gives rise to a series of isopolytungstates (paratungstates, metatungstates, etc.) which differ in the degree of aggregation; their use is also contemplated in this invention. Solvates of tungsten (VI) compounds are common (e.g. the dihydrate of sodium tungstate).

In a preferred embodiment, the tungsten (VI) compound in the pharmaceutical composition is a salt of tungstate. Specially preferred are the salts of cationic moieties selected from the group consisting of sodium, potassium, magnesium and calcium cations. The most preferred tungsten (VI) compounds are sodium tungstate (Na₂WO₄) and its dihydrate. The latter is commercially available. Sodium tungstate dihydrate is a white, odorless salt with fine and crystalline texture, and it is easily dissolved in water.

The product can be administered via any conventional oral delivery system, the tablet format being the preferred one. This system has been used in humans enroled in the Phase I of clinical trials of this compound for the treatment of obesity/overweight in non-diabetic humans (EP 755.681-A).

Another aspect of the present invention relates to the use of a compound formed by tungsten (VI) and a pharmaceutically acceptable chemical moiety, or of a solvate of said compound, for the preparation of a pharmaceutical composition for the prophylactic and/or therapeutic treatment of a neurodegenerative disorder in a mammal, including a human. In a particular embodiment the neurodegenerative disorder is a tauopathy such as Alzheimer's disease, frontotemporal dementia with parkinsonism linked to chromosome 17, progressive supranuclear palsy, amyotrophic lateral sclerosis/parkinsonism-dementia complex of Guam, Pick's disease, corticobasal degeneration, and Argyrophilic grain disease. In another particular embodiment the neurodegenerative disorder is schizophrenia.

The invention also relates to a method of treatment and/or prophylaxis of a mammal, including a human, suffering from or being susceptible to neurodegenerative disorders such as those mentioned above, in particular to Alzheimer's disease or schizophrenia, said method comprising the administration to said patient of a therapeutically effective amount of a compound formed by tungsten (VI) and a pharmaceutically acceptable chemical moiety, or of a solvate of said compound, including hydrates, together with pharmaceutically acceptable diluents or carriers.

The therapeutic treatment of tauopathies that derives from this invention is a novel approach and, over treatments previously proposed in the art, it involves several striking advantages: it targets a GSK3; specificity, it reduces the abnormal hyperphosphorylation of a neural specific protein, tau; efficacy, it exerts its action in the short term; lack of toxicity, Phase I of clinical trials has been made; and low price.

Additional objects, advantages and features of the invention will become apparent to those skilled in the art upon examination of the description or may be learned by practice of the invention. Throughout the description and claims the word "comprise" and variations of the word, are not intended to exclude other technical features, additives, components, or steps. The disclosure in the abstract of this application is incorporated herein as reference. The following examples and drawings are provided by way of illustration, and they are not intended to be limiting of the present invention.

### BRIEF DESCRIPTION OF THE DRAWINGS

FIG. 1 is a graphical illustration of the effect of tungstate on GSK3-β and tau phosphorylation on SH-SY5Y neuroblastoma cells.
FIG. 2 is a graphical illustration of the effect of tungstate on the phosphorylation of tau in brains from healthy and insulin-resistant rats.
FIG. 3 is a graphical illustration of the effect of tungstate on the phosphorylation of tau from brains obtained from STZ-diabetic rats.

### DETAILED DESCRIPTION OF PARTICULAR EMBODIMENTS

### Detailed description of the figures

The results are, in part, set out graphically in the accompanying figures:
In FIG. 1 the effect of tungstate (W) on GSK3-β and tau phosphorylation on SH-SY5Y neuroblastoma cells is shown. The data correspond to the mean values and the error bars represent the standard deviation of three independent experiments. A) SH-SY5Y neuroblastoma cells were incubated for 10 minutes in the presence (+) or absence (-) of 1 mM sodium tungstate and their protein extracts were then probed by Western blot with an antibody that recognizes GSK3-β phosphorylated at Ser-9 and total GSK3-β. GSK3-β phosphorylation on Ser-9 causes its inactivation (D.A: Cross et al., Nature, vol. 378, pp.785-789). The diagram shows the ratio between Ser-9 phosphorylated GSK3-β (GSK3-β-pS9) and the total amount of GSK3-β. B) SH-SY5Y cells were incubated with increasing concentrations of tungstate and the degree of tau phosphorylation was determined by Western blot with the tau-1 antibody, which recognizes tau when the serines 198, 199 or 202 are in unphosphorylated form. The diagram shows the ratio between unphosphorylated (tau1) and the total amount of tau (7.51). C) SH-SY5Y cells were incubated for 10 minutes in the presence (+) or absence (-) of 1 mM sodium tungstate (W) and the degree of tau phosphorylation was determined by Western blot with the AT180 antibody, which recognizes tau when threonine 231 is in phosphorylated form. The diagram shows the ratio between phosphorylated (AT180) and the total amount of tau (7.51). The total amount of tau protein was determined using the 7.51 antibody.
In FIG. 2 the effect of tungstate (W)on the phosphorylation of tau in brains from healthy and insulin-resistant rats is shown. A) Western blot analysis of the phosphorylation of GSK3 (left) and tau (right) from brain extracts of healthy rats intracranially injected with saline (-) or 0.1 mM tungstate (+). Phosphorylation was measured with antibodies against Ser-9 phosphorylated GSK3-β (pS9), and tau phosphorylated at threonine 231 (AT180) or unphosphorylated at serines 198, 199 and 202 (tau-1). The total amount of GSK3 and tau was determined through the interaction with antibodies raised against GSK3 and against tau (7.51), respectively. B) Western blot analysis of the phosphorylation in brain of GSK3-β (left) and tau (right) in control (Ctrl) and insulin-resistant (IR) rats. Phosphorylation was measured using the antibody that reacts with Ser-9 phosphorylated GSK3-β (pS9-GSK3-β) or with the tau-1 and AT180 antibodies. The total amounts of GSK3 and tau were determined as in A. C) Insulin-resistant rats were injected intracranially with saline (-) or 0.1 mM tungstate (+), their brains were then isolated and homogenised, and Western blots of the brain proteins were performed with the tau-1, and AT180 antibodies. The total amount of tau protein was determined using the 7.51 antibody. A decrease in the interaction with AT180 and an increase for that of tau-1 were observed
In FIG. 3 the effect of tungstate (W) on the phosphorylation of tau from brains obtained from STZ-diabetic rats is shown. A) Western blot analysis of Ser-9 phosphorylation of GSK3 in rat brain extracts from control healthy rats intraperitoneally injected with saline (Ctrl) or tungstate (W) (50 mg/kg). The diagram shows the ratio between pS9-GSK3 and the total amount of protein (GSK3-β). B) as in A), STZ-diabetic rats intraperitoneally injected with 50 or 75 mg/kg of tungstate. C) Western blot analysis of the phosphorylation of tau at the site recognized by the tau-1 antibody in brain extracts from control healthy rats intraperitoneally injected with saline (Ctrl) or tungstate (W) (50 mg/kg). The diagram shows the ratio between tau recognised by the tau-1 antibody and the total amount of tau protein recognised by the 7.51 antibody. D) as in C), STZ-diabetic rats intraperitoneally injected with 50 or 75 mg/kg of tungstate.The data correspond to mean values and the error bars represent the standard deviation of three to five independent experiments.

### EXAMPLES

### Antibodies and reagents

Sodium tungstate dihydrate was obtained from Carlo Erba (Milan, Italy). The following anti-tau antibodies were used: 7.51 (M. Novack et al., Proc. Natl. Acad. Sci. USA 1991, vol. 88, pp. 5837-5841) (a kind gift from Dr. C. Wischik, University of Aberdeen, Aberdeen, UK), AT-180 (Innogenetics, Gent, Belgium), and tau-1 (Chemicon, Temecula, CA). Antibody AT-180 recognizes tau when threonine 231 is phosphorylated. Antibody tau-1 recognizes tau when it is dephosphorylated at serines 198, 199 and 202 and the 7.51 antibody recognizes total tau protein. The numbering of the tau epitopes is given according to the longest human tau isoform of 441 amino acids. The other antibodies used were: phospho-GSK3-β (Ser-9) and anti-GSK3 from Cell Signaling, (Beverly, MA). Enzymes and biochemical reagents were from Sigma-Aldrich (St. Louis, MO), unless otherwise indicated. All other chemicals were of analytical grade.

### Western Blot analysis

The analysis of the degree of the phosphorylation of tau and GSK3 was performed by Western blot with the antibodies mentioned above. Cells were homogenized at 4°C in 50 mM 4-(2-hydroxyethyl)piperazine-1-ethanesulfonic acid (HEPES), pH 7.4, 10 mM ethylenediaminetetraacetic acid (EDTA), 0.1 % 4-(1,1,3,3-tetramethylbutyl)phenyl-polyethylene glycol (triton X-1 00), 20 mM NaF, 0.1 mM sodium orthovanadate, 1 mM phenylmethanesulphonylfluoride, 10 µg/ml Isovaleryl-Val-Val-4-amino-3-hydroxy-6-methylheptanoyl-Ala-4-amino-3-hydroxy-6-methylheptanoic Acid (Pepstatin A); and 10 µg/ml aprotinin. The cell lysates were centrifuged at 10,000 g for 30 min at 4°C and then heated at 100°C for 5 min in electrophoresis sample buffer. The protein concentration was determined using the BCA protein assay (Pierce, Rockford, IL). Proteins were fractionated by sodium dodecylsulfate-polyacrylamide gel electrophoresis (SDS-PAGE) on 10% gels and transferred to nitrocellulose membranes (Schleicher & Schuell Bioscience, Dassel, Germany). After blocking non-specific protein binding to the membranes with 0.05% polyethylene glycol sorbitan monolaurate (Tween 20) and 5% non-fat dry milk in phosphate buffered saline (PBS), the membranes were incubated overnight with the primary antibodies in blocking buffer at 4°C. The proteins recognized by the antibodies were visualized using enhanced chemiluminiscence (Perkin Elmer Life Sciences Boston, MA) after incubation with horseradish peroxidase (HRP)-linked secondary antibodies (Dako A/S Glostrup, Denmark). Densitometric analysis of labelled protein bands was performed with GS-710 software (Bio-Rad, Hercules, CA) and phospho-protein levels were normalized by calculating the phospho-dependent/phospho-independent protein ratio.

### EXAMPLE 1: Effect of tungstate on the phosphorylation of GSK3-β at Ser-9 in the neuroblastoma cell line SH-SY5

To examine whether tungstate influences neuronal cells, the effect of sodium tungstate dihydrate on the inhibitory phosphorylation of GSK3-β at Ser-9 in the neuroblastoma cell line SH-SY5Y (ref.: 94030304 of the European Collection of Cell Cultures, Salysbury, Whiltshire, Great Britain) was analyzed. Human neuroblastoma SH-SY5Y cells were grown in Dulbecco's Modified Eagle Medium (DMEM; Invitrogen-Gibco, Carlsbad, CA) supplemented with 10% foetal bovine serum (FBS; Invitrogen-Gibco), 2 mM glutamine, and 50 µg/ml gentamycin. The day before the experiment, the cells were plated in multiwell plates with NeuroBasal (NB) serum-free medium (Invitrogen-Gibco). Sodium tungstate dihydrate was dissolved in double distilled water and added to the culture medium at a final concentration of 0.1, 1 or 5 mM and cells were incubated for 5 or 10 minutes. Control cells were incubated with the same volume of vehicle. Exposure of Human neuroblastoma SH-SY5Y cells to sodium tungstate significantly increased the Ser-9 phosphorylation of GSK3-β (FIG. 1A), indicating that this enzyme was inactivated by the treatment.

Furthermore, it was tested whether the inhibitory action of tungstate on GSK3 was correlated with a decrease of tau phosphorylation at the consensus sites modified by this kinase. Thus, the phosphorylation of tau in SH-SY5Y cells exposed to tungstate was analyzed. An antibody that recognizes the tau protein but that is sensitive to phosphorylation of residues modified by GSK3 in Western blots was assayed. The tau-1 antibody interacts with tau when serines 198, 199 or 202 are unmodified but not when these serine residues are phosphorylated. A significant increase in the immunoreactivity with the tau-1 antibody was observed (FIG. 1 B), suggesting that exposure to tungstate impaired the phosphorylation of serines 198, 199 or 202 in the tau protein. In addition, a decrease in the phosphorylation of the epitope recognized by another antibody (AT-180) that recognizes a GSK3 phosphorylation dependent epitope in tau, was also observed in cells treated with tungstate (FIG. 1C).

These results show that tungstate stimulates the phosphorylation of GSK3-β on Ser-9 and inhibits tau phosphorylation at certain sites modified by this kinase in neural cells.

### EXAMPLE 2: Effect of sodium tungstate on the phosphorylation of tau in vivo

To study the effects of tungstate on the phosphorylation of tau in vivo, the degree of phosphorylation of this protein and that of GSK3-β at Ser-9 was first analyzed in brains of untreated and tungstate-treated healthy Wistar rats. Male Wistar rats weighing 220-240 g (IFFA CREDO, L'Arbresse, France) were caged individually in a 12:12-h light-dark cycle under controlled temperature and humidity. We did not observe significant changes in phosphorylation of either tau or GSK3 in the presence or absence of tungstate in either group (FIG. 2A). These results are concordant with previous data that show that tungstate is not active when assayed on healthy animals.

Then, the effects of tungstate were tested in a model of insulin resistance in which it is pharmacologically active: obese rats induced by a fat-rich diet. The rats were fed either a control diet (8% calories as fat, type A04 from Panlab, Barcelona, Spain), or a high fat diet, in which 65 % of the calories were in the form of fats (cf. M. Claret et al., Obes Res 2004, vol. 12, pp.1596-1603), to induce insulin resistance. After 30 days under these dietary conditions, rats were anaesthetized and 5 µl of saline or a 110 nM solution of sodium tungstate in saline was injected intraventricularly as described ("Tungstate Decreases weight gain and adiposity in obese rat through increased thermogenesis and lipid oxidation", M. Claret et al, Endocrinology, vol. 146, pp. 4362-4369). After 24 hours, rat brains were removed and the cortexes were isolated and homogenized in phosphate buffered saline. The protein extracts were probed with anti-tau and anti-GSK3- β antibodies by Western blot.

The levels of GSK3-β phosphorylated at Ser-9 were reduced in brains from insulin-resistant rats when compared with healthy lean animals (FIG. 2B left). This finding indicates that this kinase is more active in the insulin-resistant rats. This should result in an increase in tau phosphorylation in this group of animals as this protein is an excellent substrate of GSK3. Indeed, there was an increase in AT180 antibody-binding to tau and a decrease in the association of the tau-1 antibody in obese rats when compared to non-obese controls (FIG. 2B right). Tungstate treatment decreased the phosphorylation of tau at the epitope recognized by the AT180 antibody and increased the amount of protein recognized by the tau-1 antibody (FIG. 2C). These results are consistent with the data obtained from cultured neural cells: tungstate treatment reduced GSK3-dependent tau phosphorylation.

In addition, we tested the effects of tungstate on a model of type-1 diabetes, the streptozotocin (STZ)-diabetic rat (cf. A. Junod et al., J. Clin. Invest. 1969, vol. 48, pp. 2129-2139). Diabetes was confirmed by the determination of glycemia from the tail vein (Glucometer Elite, Bayer, Barcelona, Spain). A total of 3 experimental and 3 control rats were used (plus or minus tungstate). Tungstate solution was injected intraperitoneally I (50 or 75 mg/kg) to STZ-diabetic rats.

Again no changes were observed in the phosphorylation of GSK3 at Ser-9 or tau phosphorylation at the site recognized by the tau-1 antibody, in either untreated or tungstate-treated healthy animals (FIGS. 3A and C). However, in the STZ-diabetic animals, tungstate treatment increased Ser-9 phosphorylation of GSK3-β and decreased tau phosphorylation at the site recognized by the tau-1 antibody (FIGS. 3 B and D).

These results show that sodium tungstate decreases the phosphorylation of tau in insulin-resistant and in diabetic rats. All experiments were performed in accordance with the principles of laboratory animal care (European Community and local government guidelines), and the protocols were previously approved by the Animal Research Committee of the University of Barcelona.

## Claims

1. A pharmaceutical composition comprising an effective amount of a compound formed by tungsten (VI) and a pharmaceutically acceptable chemical moiety, or of a solvate of said compound, in combination with pharmaceutically acceptable excipients or carriers, for use as a prophylactic and/or therapeutical agent against a neurodegenerative disorder in a mammal, including a human.

2. The pharmaceutical composition according to claim 1, wherein the neurodegenerative disorder is a tauopathy.

3. The pharmaceutical composition according to claim 2, wherein the tauopathy is selected from the group consisting of Alzheimer's disease, frontotemporal dementia with parkinsonism linked to chromosome 17, progressive supranuclear Palsy, amyotrophic lateral sclerosis/parkinsonism-dementia complex of Guam, Pick's disease, corticobasal degeneration, and Argyrophilic grain disease.

4. The pharmaceutical composition according to claim 3, wherein the tauopathy is Alzheimer's disease.

5. The pharmaceutical composition according to claim 1, wherein the neurodegenerative disorder is schizophrenia.

6. The pharmaceutical composition according to any of the claims 1-5, wherein the compound of tungsten (VI) is a salt of tungstate comprising a pharmaceutically acceptable cationic moiety.

7. The pharmaceutical composition according to claim 6, wherein the cationic moiety is selected from the group consisting of sodium, potassium, magnesium and calcium cations.

8. The pharmaceutical composition according to claim 7, wherein the compound of tungsten (VI) is sodium tungstate.

9. The pharmaceutical composition according to claim 7, wherein the compound of tungsten (VI) is sodium tungstate and said solvate is the dihydrate.

10. Use of a compound formed by tungsten (VI) and a pharmaceutically acceptable chemical moiety, or of a solvate of said compound, for the preparation of an oral composition, for the prophylactic and/or therapeutical treatment of a neurodegenerative disorder in a mammal, including a human.

11. Use according to claim 10, wherein the neurodegenerative disorder is a tauopathy.

12. Use according to claim 11, wherein the tauopathy is selected from the group consisting of Alzheimer's disease, frontotemporal dementia with parkinsonism linked to chromosome 17, progressive supranuclear Palsy, amyotrophic lateral sclerosis/parkinsonism-dementia complex of Guam, Pick's disease, corticobasal degeneration, and Argyrophilic grain disease.

13. Use according to claim 12, wherein the tauopathy is Alzheimer's disease.

14. Use according to claim 10, wherein the neurodegenerative disorder is schizophrenia.

15. Use according to any of the claims 10-14, wherein the compound of tungsten (VI) is a salt of tungstate comprising a pharmaceutically acceptable cationic moiety.

16. Use according to claim 15, wherein the cationic moiety is selected from the group consisting of sodium, potassium, magnesium and calcium cations.

17. Use according to claim 16, wherein the compound of tungsten (VI) is sodium tungstate.

18. Use according to claim 16, wherein the compound of tungsten (VI) is sodium tungstate and said solvate is the dihydrate.
